# EUROPEAN PATENT APPLICATION

(11) **EP 0 703 541 A1**
(43) Date of publication of application: **27.03.1996**
(21) Application number: 94115042.7
(22) Date of filing: 23.09.1994
(51) Int. Cl.: G06F 19/00, H04N 1/028

(54) **Apparatus and method for aiding diagnostic analysis of medical images, particularly dental X-ray-images**

(71) Applicant: Vaisfeld, Sorin, Dr., D-97816 Lohr am Main (DE); Redik Ltd., Bat Yam (IL)
(72) Inventor: Havel, Jan, Dr., Hulon (IL)
(74) Representative: Hansmann, Axel, Dipl.-Wirtsch.-Ing.

(57) **Abstract**

The application discloses an apparatus and method for aiding diagnostic analysis of medical images, particularly dental X-ray-images, the apparatus including a holder for the image to be analysed, an illumination source and an optical viewing device, with the combination of said image holder and illumination source with an electrooptic capture device for digitizing the image and a computer for storing and handling the digital data received, a screen being provided for display of the image. The apparatus is of a modular structure for satisfying different needs.

A program unit is provided for electrical integration of the components of the apparatus. According to ome embodiment a drawer for holding the image to be analysed, the illumination source, the illumination tuner, the electrooptic capture device and the computer including the storage unit are an integrated structural unit with connection to a computer control keyboard with or without mouse and a display screen.

For use in connection with small-size X-ray-images, the electrooptic capture device is CCD-array camera capturing the entire picture at one moment. For handling large-size X-ray-images a module is provided with a high resolution line-C.C.D.camera (scanner) .

## Description

The invention (according to the preceding clause of claim 1) refers to an apparatus and method for aiding diagnostic analysis of medical images, particularly dental X-ray-images, the apparatus including a holder for the image to be analysed, an illumination source, and an optical device.

According to the common practice dental X-ray-images for diagnostic analysis thereof are regarded by the dental doctor under illumination and optical lenses may be used for magnifying the image for enhancing analysis thereof. Generally the small image sizes, over- or under- exposure and poor contrast and also the difficult manual handling are adversly affecting the diagnosis, are time consuming and do not allow to have the patient participate in the planning of medical attendance and therapy.

It is the problem underlying the present invention to provide an apparatus and a method for enhancing the diagnostic analysis of particularly dental X-ray-images for making best possible use of any details included in such images for the planning of medical attendance and therapy.

The solution of the problem is characterized in claim 1 of the present invention.

Further details of the invention and the method are claimed in subsequent claims.

The invention according to a simple form resides in the combination of said image holder and illumination source with an electro-optic capture device for digitizing the image, a computer for storing and handling the digital data received, a screen being provided for display of the image. Preferably a program unit is provided for electrical integration of the components of the apparatus.

The apparatus according to the foregoing invention enables the medical doctor an immediate display of the X-ray-image in a magnified size and with possible enhancement of the details of the X-ray-image by digital treatment facilities, at the same time allowing an easy digital storing of the images for future retrieval and possibly even comparison between different stored images.

According to a prefered embodiment of the invention a drawer for holding the image to be analysed, the illumination source, the illumination tuner, the electro-optic capture device and the computer including the storage unit are an integrated structural unit, preferably including the display screen also and with connection to a computer control keyboard with or without mouse.

The apparatus of the foregoing type permits an easy capturing and storing of particularly the small dental X-ray-films including periabical, bait etc., and it constitutes a structurally unitary basic equipment permiting the medical doctor an enhanced analysis of the X-ray-pictures.

With respect to the prior art it has to be noted that the illumination sources, illumination tuners, picture-digitizing apparatuses and computers available on the market as separate or partly combined units are not suited for easy handling the dental X-ray-films but rather would involve time consuming work for installing the equipment, tuning the equipment and handling the X-ray-images in a way which is beyond the possibilities in medical and dental practice.

According to a further development of the invention the unitary structural unit according to the foregoing claims has a drawer for loading small size dental X-ray-images and an integrated screen for display thereof and is electrically integrated with a supplemental module having a drawer for large-size X-ray-images, particularly panoramic images, and a large external screen for display thereof.

According to a particularly simple embodiment of the invention for use in connection with small-size X-ray-images the electro-optic capture device is a CCD-array camera capturing the entire picture at one moment, and the electrical inte-gration of the apparatus being designed for immediate display on the screen under control of the manual illumination tuner.

Preferably the apparatus in addition to the internal working storage is equipped with a structurally integrated floppy disc-drive and/or an integrated magneto-optical drive.

According to a further development a module for handling large-size X-ray-images is provided with a high resolution line CCD-camera. According to a further development the apparatus is provided also with an archives unit.

Preferably the apparatus is under the control of a data processing program according to the commercially available "WINDOWS"(TM)-type).

For specific purposes the apparatus may be provided with a connection to external computer equipment.

Alternative embodiments of illumination are characterized in the subsequent claims.

According to a further development of the invention the apparatus is provided with programmed equipment for translating different grey-levels of the X-ray-image into different types or levels of colour or brightness or contrast. This feature based on a detailed analysis of the different grey-levels appearing in X-ray-images is greatly facilitating to the dental doctor the recognition of pathologic features like cystes, caries, bone structure etc. for developing optimum curing strategies thereof.

The invention thus also is residing in a method for X-ray-diagnostics by electronically capturing an X-ray-image and characterized by computerized digitizing the image and translating the different grey-levels of the X-ray-image into different types or levels of colour, bright-ness or contrast for display and observation during the diagnosis.

According to a further development of the invention the computer of the apparatus is provided with a soundboard permitting operating the computer with voice orders and storage of voiced information.

Embodiments of the present invention are described with reference to the attached drawings.
- Fig. 1: shows a simple embodiment (ready mini cam) of the invention including a compact optical read-out unit for dental X-ray-images having a structural integrated unit with a drawer for holding the image, an illumination source, a manual light tuner, scanner, computer board and connection to an external display screen,
- Fig. 2: shows a further developed embodiment with integrated display screen,
- Fig. 3: shows a further developed embodiment including in addition to the Fig. 2-embodiment a floppy disc-drive and loud speaker;
- Fig. 4: shows a supplemental module provided with a drawer for large-size X-ray-images, high resolution optical scanner and digitizing and archivating equipment.
- Fig. 5: shows a system combined from a unit for handling small X-ray-images with integrated display screen and the supplemental module for handling large-size images and connected to an external large colour monitor and computer keyboard and mouse.
- Fig. 6A: is a bloc and circuit diagram of connection of components of the system according to fig. 5.
- Fig. 6B: shows several block diagrams according to the different embodiments.
- Fig. 7: is a showing of an image editor displaying in comparison an original X-ray-image and a (possibly coloured) update thereof, inclusive a menue-line for control of the apparatus.
- Fig. 8: is the showing of an archive card to be used in connection with the present system.

The drawings illustrate different embodiments of an apparatus and a method also for aiding diagnostic analysis of medical images, particularly dental X-ray images.

The several embodiments of the invention are based on a modular system and according to the components used and their combination different needs may be served. According to the modular system it is possible also to start with a more restricted system, which then is completed afterwards.

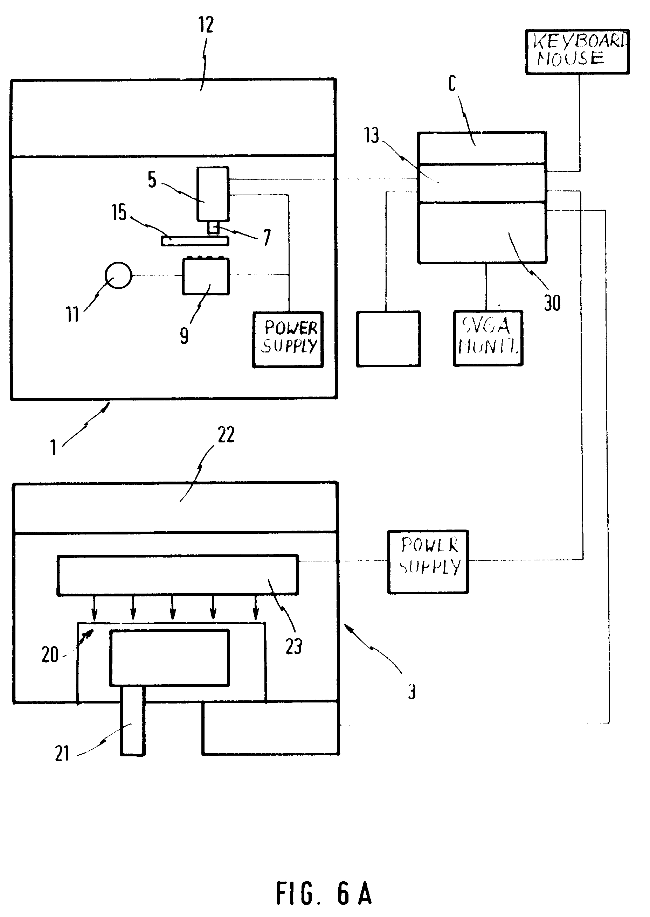

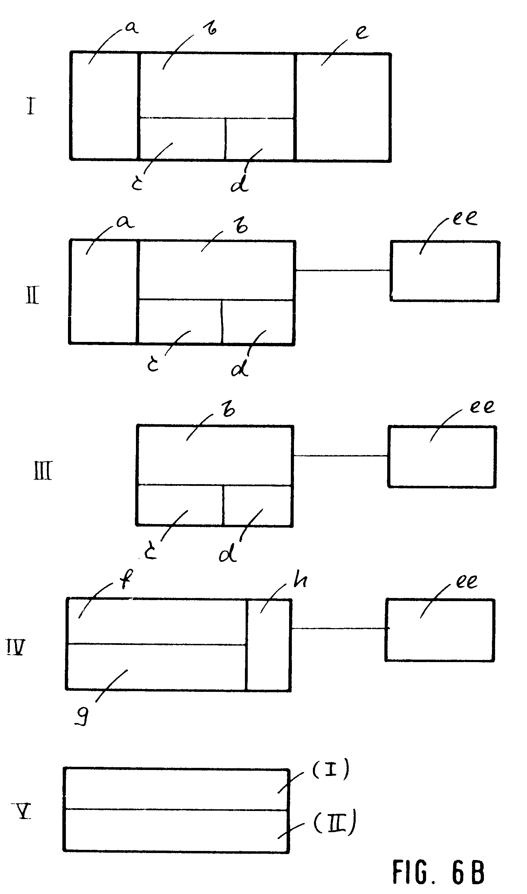

Fig. 6B is a comparative showing of different embodiments of the invention, and fig. 6A shows a block and circuit diagram of the most completed system.

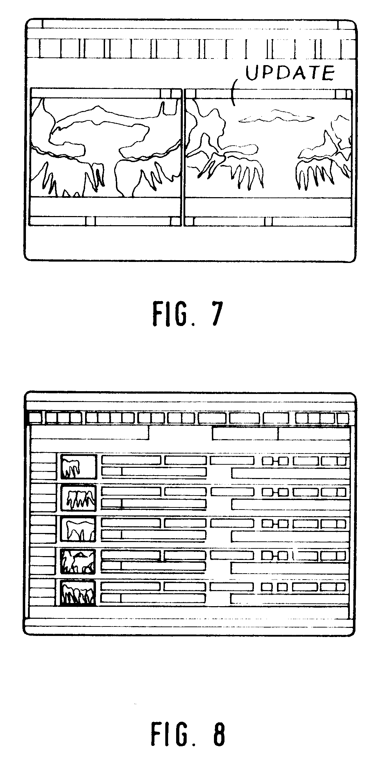

The several embodiments are controlled by a software handled in accordance with the WINDOWS(TM)-system. This is illustrated in fig. 7 and 8. The menues and symbols help the user in quickly performing the digital X-ray-diagnosis.

When the X-ray images have been read in these can be handled automatically or optimized manually. Functions as enlargement of partial areas, tuning brightness and contrast, varying grey levels, positive/negative presentation, optimizing, colouring and comparing different X-ray images help particularly the dental doctor in the diagnosis. Even X-ray images of weak contrast or overexposed can be used. The showing on the display screen allows explanation of the diagnosis to the patient also.

A practical function is the length measuring at the screen e.g. for endodontic or paradontal treatment.

The text editor allows the integration of comentaries and diagnostic results along with indication arrows and graphic symbols and the storage thereof. As illustrated in fig. 7 and 8 the X-ray images and other relevant information can easily be stored in an archive. Upon continuing the attendance an immediate access to the X-ray images and diagnostic results is possible.

The software makes use of an effective compression algorithm for compressing the existing data. Thus a large number of pictures and patient cards can be stored with usual sold disc storages.

For providing the system according to the present invention and its components the invention is in part relaying on known electronic elements and equipment which are adapted and combined in a unique manner for attaining the results described. For controlling and operating the system and the components thereof, the invention also makes use in a unique manner of known software with specific adaption and incorporation into the system.

In the following the features of the system are described in more detail.

As regards the hardware of the most complete system as shown in fig. 6A the system is based on a computer C to operate and control of two electro-optic units 1 and 3, respectively, capture and store the various kinds of dental films. These two electro-optic units differ one from the other by the size of X-ray films each can handle. Unit 1 is for handling the group of small dental X-ray films including: periapical, bait, etc. Unit 3 is for handling the group of big X-ray films (panoramic, status).

### Details of electro-optic unit 1 for small size X-ray films are as follows:

This unit 1 is based on a CCD array camera 5, a lens 7 which images the X-ray film on the CCD surface and an illumination module 9 based on an array of LEDs (Light Emitted Diodes). The light intensity emitted by the LED array module 9 is controlled with the aid of an intensity tuner 11 by the user to get the optimum picture on the screen before it is captured by the electro-optic capture device 12 and digitized. The image is transferred to the computer by a frame grabber, which captures and digitizes the video output of the camera C.

The illumination source module 9 for the small X-ray is provided with an array of sensitive light-emitting diodes (LEDs) permitting dynamic intensity tuning. To get the optimum X-ray picture before it is captured and digitized.

In contrast to the use of fluorescent light source or the likes of it the LED-array provides for a particularly space saving structure and eliminates undesired heat development. The dynamic intensity tuning ensures optimal picture quality on the screen before it is captured and digitized.

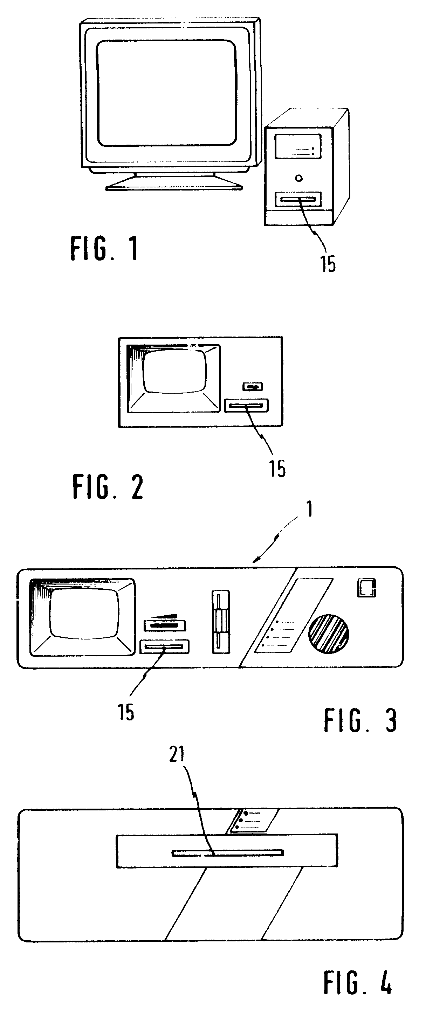

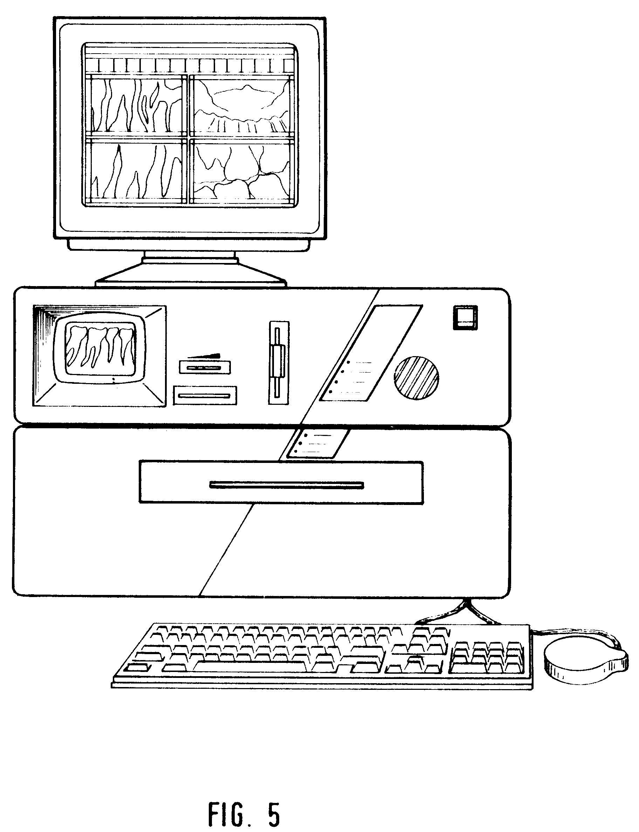

A film loader 15 may be in the form of a drawer as illustrated in Fig. 1 - 5.

### The details of the electro-optic unit 3 for large size X-ray films are as follows:

The large size X-ray films which are loaded into the system with the aid of loader 21 (see fig. 4 also), is captured to the computer by a scanner based on line CCD camera 20. The image of the film is captured line by line while it is scanned by the electro-optic head of the scanner 22. In this way the horizontal resolution is determined by the resolution of the line CCD (number of pixels per unit length). The vertical resolution on the other hand is determined by the size of the pixel convoluted with the resolution of the motion. One of the main disadvantages of scanners may be the motion involved with the imaging. This motion should be in a very precise increments. The precision is determined by the vertical resolution which is in the present case few tenth of microns.

The illumination of the object should be very stable referring to the CCD head during the scanning. This is particularly true in transparencies like X-ray films since the intensity fall on the CCD surface is directly proportional to the intensity on the light source multiplied by the density of the film at each imaged point. A unic solution has been applied in the present system to the problem of light stability. Instead of line light source which moves synchronically with the electro-optic head. The light source is a two dimensional high intensity fluorescent light source 23 which covers all the scanned area. One of the main problems with fluorescent lights is their flickering in the frequency of the power-supply (100-120 Hz or 50-100 KHz in the case of electronic power-supply). In the present system that problem is solved by an electronically modified power-supply providing a smoothed DC electric supply.

The illumination source 23 for the large X-ray umages device is based on a two-dimensional high intensity fluorescent light source which covers the whole scanned area and a special modification in the light power supply make it possible to get a smooth DC electric supply. This method overcomes the main disadvantage existing in commercial scanners. The image of the film involved is captured line by line while it is being scanned by the electro-optic head of the scanner. In the known method the light source is moving according to the movement of the line-CCD camera. The vertical resolution of the image is determined by the size of the pixel convolute with the resolution of the motion.

This method of motion of the light source is one of the main disadvantages because it should be in very precise increments. The precision is determined by the vertical resolution which is in the case of the invention a few tenth of microns. The illumination of the object should be very stable referring to the CCD head during the scanning.

This is particularly true in transparencies like X-ray films since the intensity fall on the CCD surface is directly proportional to the intensity on the light source multiplied by the density of the film at each imaged point.

In contrast to the above, the light source according to the invention is not moving with the electro-optic head and as a result it is stable and uniform.

Another problem overcome is the flickering of fluorescent lights in the frequency of the power supply (100-120 Hz or 50-100 KHz in the case of electronic power supply).

As aforementioned the solution is owing to the use of an electronic modification in the power supply that results in the avoidance of flickering.

With the embodiment for capturing the large size X-ray images brightness is automatically software controlled in accordance with a preview scanning of the X-ray image.

The computer C is a module built as a standard PC mother-board with several computer boards on it, each of which performs a particular function.

These boards include a high performance display board including an accelerator for WINDOWS (TM).

The SCSI-board 30 is used as a fast interface between the scanner and the computer for grabbing the X-ray image from the scanner to the computer memory.

The frame grabber board 13 captures and digitizes the video image output from the camera and stores it in the computer memory.

A sound board enables the user to communicate with the computer by voice instead of screen or keyboard. The user can record remarks during the treatment and retrieve them any time. By means of A.S.R. (Automatic Speech Recognition) the system with the sound board and particular software enables the user to operate the computer with voice orders instead of keyboard or mouse.

A LAN-board (local area network board) can connect between the system and the rest of computers in a clinic.

Also a fax/modem can be connected for communication and data transfer between a clinic, insurance companies, other clinics or the like.

As regards the software, the system is based on Relational Data Base as a complete concept of the system. The system is based on WINDOWS G.U.I. (Graphic User Interface). While keeping the concept of operating the system with minimum interactions of the user with the keyboard or even the mouse the software is built of two basic models to with
1. X-ray film module and
2. Clinic Management module.

The X-ray module includes several main functions:
X-ray capturing - This module is the interface between the hardware which garb the X-ray image from the CCD array 5 or the scanner 22 according the kind of X-ray film loaded to the system.

The module is connected to the diagnostic subsystem and management module so that with the image stored in the computer memory all the attributes on the X-ray film is kept and connected to the picture.
These attributes includes:
picture number, patient information, picture capturing date, treatment information as illustrated by fig. 8.

An important possibility rendered by the present invention is image processing.

After the X-ray film was captured as part of the diagnostic processing of the doctor, the image processing module is opened automatically. This module enables the doctor to perform specific image enhancement operations on the picture in order to improve the diagnostic part of the treatment. Among the many image processing function in that module the following can be mentioned:
Zooming the image from 1/20 to X100 of its actual size.
Sharpness - A feature which enables to sharpen edges in the border of the objects in the picture.
R.O.I. (region of interest) - These is ability to mark and treat a specific region of the image and emphasize a specific characteristic in that region compared to the background.
Brightness/Contrast - These is feature to turn the brightness and the contrast of the picture of R.O.I..
Pseudo Color - representation of the variances in grey levels from which the picture is built of by color mapping.
Histogram Equalization - An automatic tuning of the grey levels is the picture or R.O.I. to the full range of grae levels.

### Saving and retreiving pictures:

After the capturing or the image processing is finished the picture is stored to the electronic archive provided in the system according to the present invention. The storage is performed while compressing the original image up to 1/20 of it's original size without any loss of visual information in the picture. The archive is part of the Relational Data Base which includes the diagnostic and the management information on the pictures in the archive.

## Claims

1. Apparatus for aiding diagnostic analysis of medical images, particularly dental X-ray-images, the apparatus including a holder for the image to be analysed, an illumination source and an optical viewing device,
**characterized by**
the combination of said image holder and illumination source with an electrooptic capture device for digi-tizing the image and a computer for storing and handling the digital data received, a screen being provided for display of the image.

2. Apparatus according to claim 1,
**characterized by**
an illumination tuner.

3. Apparatus according to claim 1 or 2,
**characterized by**
a program unit for electrical integration of the components of the apparatus.

4. Apparatus according to claim 1, 2 or 3,
**characterized in that**
a drawer for holding the image to be analysed, the illumination source, the illumination tuner, the electrooptic capture device and the computer including the storage unit are an integrated structural unit with connection to a computer control keyboard with or without mouse and a display screen.

5. Apparatus according to claim 4,
characterized in that
the structural unit is including the display screen also.

6. Apparatus according to claim 5,
**characterized in that**
the unitary structural unit has a drawer for loading small-size dental X-ray-images and an integrated screen for direct display thereof and is electrically integrated with a supplemental structural module having a drawer for large-size X-ray-images, particularly panoramic images, and a preferably larger size additional external screen for display of images processed electronically for emphasizing specific features.

7. Apparatus according to any of claims 1 to 4, for use in connection with small-size X-ray-images,
**characterized in that**
the electrooptic capture device is CCD-array camera capturing the entire picture at one moment, the electrical integration of the apparatus being designed for immediate display on the screen under control of the manual illumination tuner.

8. Apparatus according to any of the preceding claims,
**characterized in that**
the apparatus in addition to the internal working storage is equipped with a structurally integrated floppy disc-drive and/or an integrated magneto-optical drive.

9. Apparatus according to claim 6 or the following,
**characterized in that**
the module for handling large-size X-ray-images is provided with a high resolution line-C.C.D.camera (scanner) .

10. Apparatus according to any of the preceding claims,
**characterized in that**
the computer is provided also with an archives unit.

11. Apparatus according to any of the preceding claims,
**characterized by**
the control by a data processing program according to the commercially available "Windows"(TM)-type.

12. Apparatus according to any of the preceding claims,
**characterized by**
a connection to external computer equipment or net-work.

13. Apparatus according to any of the preceding claims,
characterized in that the apparatus is provided with programmed equipment for translating different grey-levels of the X-ray-image into different types or levels of colour or brightness or contrast.

14. Apparatus according to any of the preceding claims,
**characterized in that**
the computer of the apparatus is provided with a soundboard permitting operating the computer with voice orders and storing of voiced information.

15. Apparatus according to claim 9,
**characterized by**
means for preview scanning and automatic brightness control in accordance with the preview result.

16. Apparatus according to claim 9 or 15,
**characterized by**
a high intensity fluorescent light source covering all the scanned area and connected to a smooth DC electric supply.

17. Apparatus according to particularly claim 7,
**characterized by**
an illumination module based on an array of LEDs.

18. Method for X-ray-image-diagnostics,
**characterized by**
electronically capturing the X-ray-image, computerized digitizing the image and translating the different grey-levels of the X-ray-image into different types or levels of colour, brightness or contrast for display and observation during the diagnosis.
